# EUROPEAN PATENT SPECIFICATION

(11) **EP 2 124 829 B1**
(45) Date of publication and mention of the grant of the patent: **15.01.2014**
(21) Application number: 08701832.1
(22) Date of filing: 17.01.2008
(51) Int. Cl.: A61F 2/40

(54) **REVERSE SHOULDER PROSTHESIS**
INVERSE SCHULTERPROTHESE
PROTHÈSE D'ÉPAULE INVERSÉE

(30) Priority: 18.01.2007 GB 0700940
(43) Date of publication of application: 02.12.2009
(73) Proprietor: THE UNIVERSITY OF NEWCASTLE UPON TYNE, Newcastle Upon Tyne NE1 7RU (GB)
(72) Inventor: JOHNSON, Garth, Co Durham DH8 OHT (GB); KONTAXIS, Andreas, Newcastle upon Tyne NE1 4XB (GB); WALLACE, Angus, Nottingham NG7 2UH (GB)
(74) Representative: Atkinson, Jennifer
(86) International application number: PCT/GB2008/000154
(87) International publication number: WO 2008/087415

(56) References cited:
- EP-A- 1 064 890
- EP-A- 1 607 067
- EP-A- 1 607 068
- EP-A- 1 607 069
- EP-A- 1 607 070
- FR-A- 2 860 427

## Description

The present invention relates to a shoulder prosthesis, and in particular to a scapula component and a humerus component which together form a reverse shoulder prosthesis.

Reverse (or inverse) shoulder prostheses are known. Such prostheses are termed reverse as they reproduce the scapula-humerus interaction in reverse. In a normal shoulder, a spherical element located on the proximal end of the humerus interacts with the glenoid cavity on the scapula. In a reverse shoulder prosthesis the spherical element is located on the scapula and the cavity, which interacts with the spherical element, is located on the proximal end of the humerus. Reverse shoulder prostheses are typically used in the event of serious muscular degeneration of the shoulder, particularly of the rotator cuff muscles. The degeneration of such muscles causes a prevalent action by the deltoid which tends to draw the humerus upwards and raises the risk of it contacting the acromion on the scapula. To avoid this contact the shoulder joint is reversed. The reverse shoulder prosthesis provides stability to the joint and maximises mobility of the shoulder. Reverse shoulder prostheses are the most common form of prosthesis used to treat severe rotator cuff tears.

A scapula component representing the closest prior art is disclosed in EP 1 607 070 -A.

A significant problem with existing reverse shoulder prostheses is impingement of the humerus component of the prosthesis on the inferior border of the scapula. Contact with the scapula caused by the impingement may create notches in the bone. These notches may result in bone loss and loosening of the scapula component of the prosthesis. Studies have shown that in 62% of patients with reverse shoulder prostheses, notching is observed within 2 years of inserting the prosthesis. Modelling studies have shown that especially in low-level tasks, requiring only a low degree of humeral elevation, impingement can occur. There is therefore a need for a reverse shoulder prosthesis which reduces or eliminates impingement. Such a device may extend the life of the prosthetic device, reduce the risk of potential dislocation and reduce the risk of damage to the scapula.

The invention provides a scapula component for a reverse shoulder prosthesis comprising a part spherical, convex, surface which is less than a hemisphere and wherein the part spherical surface does not have a rotational axis of symmetry.

Preferred embodiments are defined in the dependent claims.

The part spherical surface may have one or more planes of symmetry. Preferably the part spherical surface only has one plane of symmetry.

Preferably the part spherical, convex, surface comprises a main circular surface portion and one or more subsidiary surface portions depending from the edge of the main surface portion.

Preferably the edge of the main circular surface portion is defined by a plane which intersects the part spherical surface.

Preferably the main circular surface portion is the area bounded or defined by the largest circle that will fit onto the part spherical surface.

The one or more subsidiary surface portions may depend from only a part, or from all, of the edge of the main circular surface portion.

The scapula component may comprise two subsidiary surfaces on opposite sides of the main surface portion. The two subsidiary surfaces may be crescent shaped.

The scapula component may comprise a third subsidiary surface which extends from the circular edge at the top of the main surface portion.

The main surface portion and the one or more subsidiary surfaces together form the part spherical surface. All the surfaces which form the part spherical surface have the same radius of curvature. The overall shape of the part spherical surface is preferably determined by where load forces will be placed on the surface of the scapular component when it is in use. An aim is to have a part spherical surface which includes only the load bearing those surfaces which would be present if the surface was hemispherical.

The transition between the main surface portion and the subsidiary surface portions may be smooth, and may form one continuous surface with a constant radius of curvature.

The part spherical surface does not have a rotational axis of symmetry. The part spherical surface may have one or more planes of symmetry.

Preferably the main surface portion of the part spherical surface has a rotational axis of symmetry.

The surface area of the main surface portion of the part spherical surface may be less than the surface area of a hemisphere. The surface area of the part spherical surface may be less than the surface area of a hemisphere.

The two most distant points on the edge of the part spherical surface may be spaced apart by less than two times the radius of curvature of the part spherical surface.

Preferably the surface area of the part spherical surface is at least about 5% less than the surface area of a hemisphere which has the same radius of curvature as the part spherical surface. Preferably the surface area of the part spherical surface is between about 5% and about 30% less, more preferably between about 20% and about 30% less, than the surface area of a hemisphere which has the same radius of curvature as the part spherical surface. Preferably the surface area of the part spherical surface is at least about 6, 7, 8, 9, 10, 11, 12, 13, 14, 15, 16, 17, 18, 19, 20, 21, 22, 23, 24, 25, 26, 27, 28, 29 or 30%, less than the surface area of a hemisphere which has the same radius of curvature as the part spherical surface.

Preferably the surface area of the main surface portion of the part spherical surface is at least about 5% less than the surface area of a hemisphere with the same radius of curvature as the part spherical surface and the main surface portion. Preferably the surface area of the main surface portion is between about 5% and about 35% less, more preferably between about 20% and about 35% less, than the surface area of a hemisphere which has the same radius of curvature as the part spherical surface and the main surface portion. Preferably the surface area of the main surface portion is at least about 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35%, less than the surface area of a hemisphere which has the same radius of curvature as the part spherical surface and the main surface portion.

Preferably the volume occupied by the portion of the scapula component with a part spherical surface is between about 5% and about 50% less than the volume that would be occupied by a hemisphere with the same radius of curvature, preferably the volume occupied is between about 10% and about 30% less.

The volume occupied by the scapula component of the invention is less than the volume a hemisphere comprising the same radius of curvature as the part spherical portion of the scapula component would occupy.

Preferably the part spherical surface defines a bearing surface which in use contacts the humerus component of a reverse shoulder prosthesis.

The part spherical surface may form the front face of the scapula component.

The part spherical surface may have a radius of curvature of between about 10 mm and about 30 mm, preferably between about 15mm and about 25mm, more preferably between about 18mm and about 22mm.

In known reverse shoulder prostheses the scapula component comprises a substantially hemispherical surface which forms the bearing surface. The requirement that the component is hemispherical has dictated the size of the scapula component that can be used in a reverse shoulder prosthesis. Insertion of the scapula component during shoulder replacement surgery is difficult, and there is little room for the surgeon to manoeuvre. Surgeons have therefore preferred to use smaller scapula components, typically with a hemispherical bearing surface having a diameter of between 36 and 38mm (a radius of curvature of 18 or 19mm). Although components with larger diameters, say 40 or 42mm (radius of curvature of 20 or 21mm) are known, the increase in size makes them difficult to position in the scapula and therefore unpopular with surgeons and much less commonly used.

However, a scapula component according to the invention in which the part spherical surface is less than a hemisphere, and is shaped to retain only those surfaces which are needed to take the bearing/forces put on the component in use, takes up less volume than a hemispherical component with the same radius of curvature. A scapula component according to the invention with a part spherical surface which has a radius of curvature of between about 18 and about 22 mm, may take up a volume which is same, or less than a conventional substantially hemispherical prostheses with a diameter of 36 or 38mm (a radius of curvature of 18 or 19mm). By way of example, a scapula component according to the invention with a radius of curvature of 21mm may be configured to occupy the same, or less, volume than a Delta^{®}III scapula component from DePuy™ with a diameter of 36mm (a radius of curvature of 18mm).

The advantage of providing a surface which is part spherical, but less than a hemisphere, is that the advantages of having a surface with a larger radius of curvature can be obtained without having to increase the overall size and/or volume of the scapula component. Having a bearing surface with a larger radius of curvature minimises the risk of impingement of the underlying bone, as the larger radius of the bearing surface of the scapula component drives the cup on the humeral component away and therefore reduces the risk of impingement.

Scapula components according to the invention may therefore be smaller, than known hemispherical components with the same radius of curvature, and may therefore be easier to insert during surgery.

Preferably the scapula component comprises a mounting means to mount the scapula component either directly or indirectly to the scapula.

The mounting means may be a hole or a screw or any other suitable fixation means which extends from the front of the scapula component to the back of the scapula component. Preferably the mounting means has a longitudinal axis.

Preferably the main surface portion of the part spherical surface of the scapula component has a rotational axis of symmetry that is offset at an angle from the longitudinal axis of the mounting means. Preferably the two axes are offset by an angle of between about 5 degrees and about 45 degrees, preferably the two axes are offset by an angle of between about 10 and about 30 degrees. Preferably the two axes are not laterally displaced.

The benefit of offsetting the rotational axis of symmetry of the main surface portion of the part spherical surface, relative to the longitudinal axis of the mounting means, is that it allows the part spherical surface of the scapula component to be more easily positioned for contact with a humeral component when in use.

The surface area of the part spherical surface may be less than the surface area of a hemisphere with the same radius of curvature due to the removal of material at the base of the hemisphere. The part of the hemisphere that is missing in the part spherical surface would not have been load bearing if it had been present in the scapula component of the invention.

In addition to the part spherical surface the scapular component may also comprise a part cylindrical surface. Preferably the part spherical surface and the part cylindrical surface are on the front of the scapular component.

The part cylindrical surface may extend from the back of the part spherical surface. The part cylindrical surface does not form part of the bearing surface of the scapula component. The part cylindrical surface may be included on the scapula component to assist in fixation of the scapula component to the scapula, either directly or indirectly.

The scapula component may be attached directly or indirectly to the scapula. If the component is attached indirectly a mounting member may be used. The mounting member may attach on one side to the scapula and on the other side to the scapula component. The mounting member may have a convex back surface which, in use, contacts the scapula.

The part spherical (bearing) surface of a scapula component according to the invention may be made of stainless steel, titanium, cobalt chrome, a combination thereof or any other suitable material.

Embodiments of the present invention will now be described in more detail, by way of example only, with reference to the following figures.
**Figure 1A** shows a schematic perspective view of a reverse shoulder prosthesis according to an embodiment of the invention;
**Figure 1B** shows a schematic perspective view of a reverse shoulder prosthesis according to an embodiment of the invention located in a shoulder;
**Figure 2A** shows a sketch drawing of a prior art reverse shoulder prosthesis, the arrow indicates where impingement has occurred and the bone can be seen to be notched;
**Figure 2B** is a photomicrograph of a section through a shoulder with a reverse prosthesis according to the prior art;
**Figure 2C** is a photograph of a shoulder with a reverse prosthesis according to the prior art;
**Figures 3A to 5** show perspective side and plan views of a scapula component according to a first embodiment of the invention;
**Figures 6A to 8A** show perspective side and plan views of a scapula component according to a second embodiment of the invention;
**Figure 8B** shows a perspective side view of a scapula component according to a third embodiment of the invention;
**Figures 9A to 9C** show perspective views of a scapula mounting component according to an embodiment of the invention;
**Figure 10** shows a perspective view of a scapula mounting component and a scapular component according to the second embodiment of the invention; and
**Figures 11A to 11D** show perspective views of a humerus component.

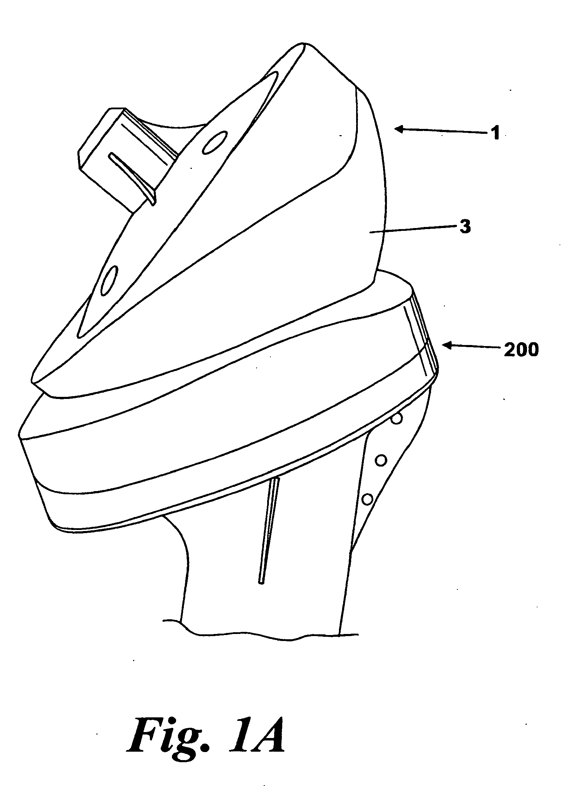

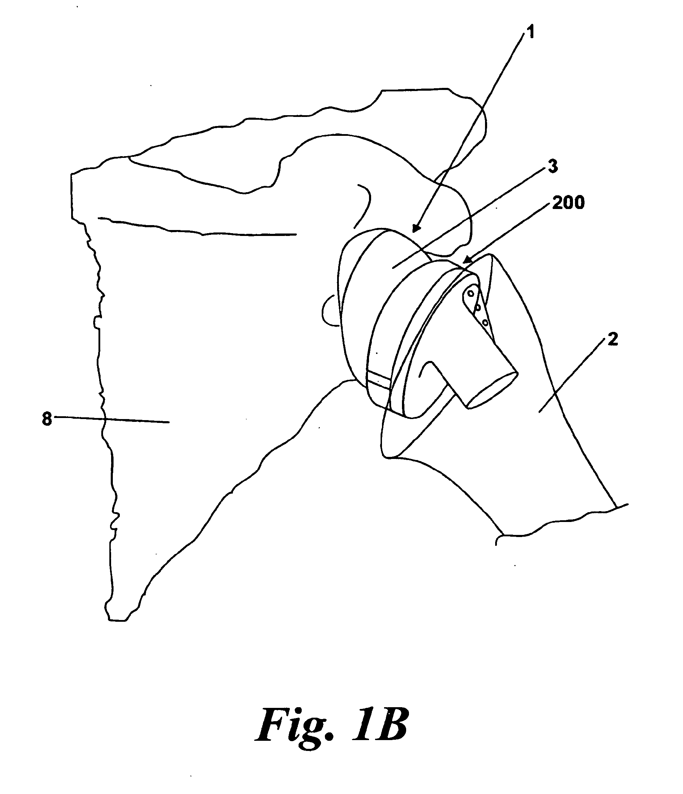

Referring to the drawings, Figure 1A shows a reverse shoulder prosthesis according to the invention comprising a scapula component 1 and a humerus component 200. The bearing surface 3 of the scapula component 1 is shown in contact with the bearing surface (not visible) of the humerus component 200. In use (Figure 1B), the scapula component 1 is mounted in the glenoid cavity on a patient's scapula 8 and the humerus component 200 is mounted to the proximal end of the patient's humerus 2. In use, the bearing surface 3 on the scapula component 1 interacts with the bearing surface of the humerus component 200 to allow the humerus 2 to move relative to the scapula 8.

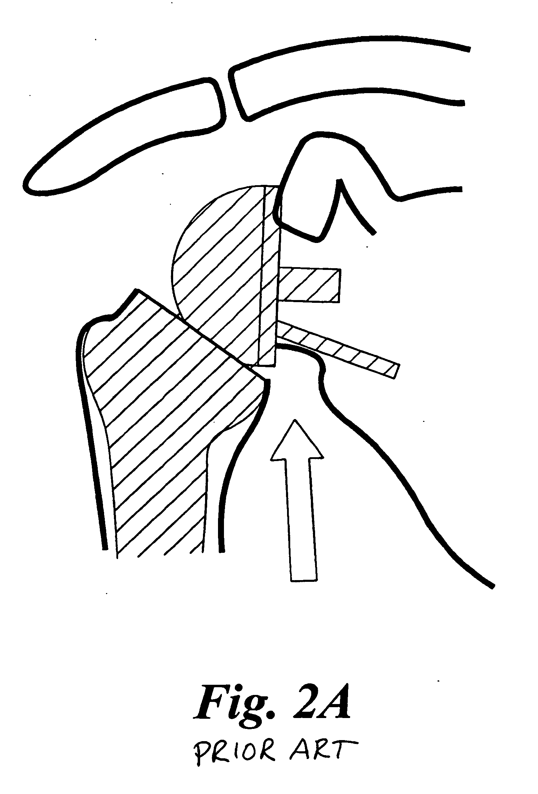

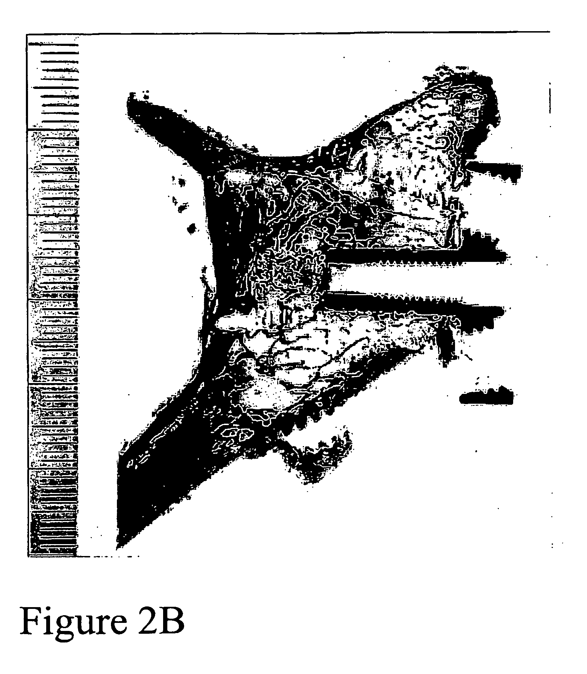

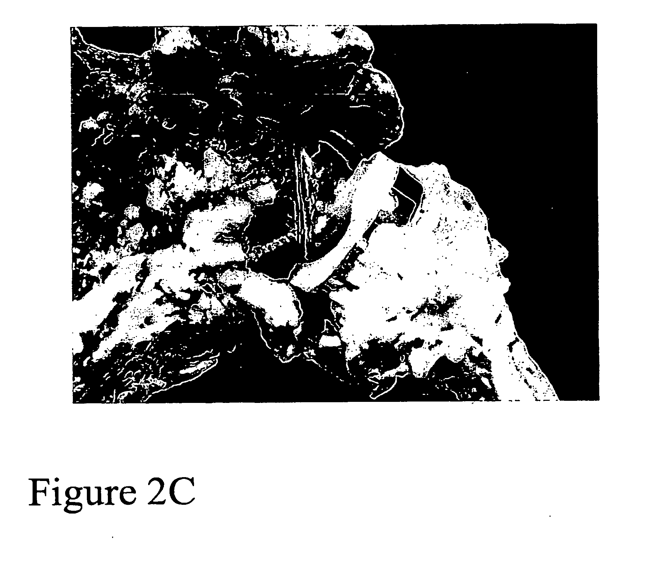

Figures 2A and 2B illustrate a prior art reverse shoulder prosthesis. Figure 2A schematically illustrates notching (indicated by the arrow) on a shoulder fitted with a reverse shoulder prosthesis. It is believed that this notching may be caused by impingement of the scapula by the humerus component of the prosthesis. Figure 2B is a photomicrograph of a section in the frontal plane through the glenoid and the scapula component of a shoulder prosthesis, illustrating the erosion of the inferior pole of the glenoid, leaving the prosthesis unsupported by bone. Figure 2C is a photograph showing the erosion around the inferior pole of the glenoid and the scapula neck. The inferior screw and the inferior part of the glenoid base plate (mounting portion) of the scapular component are denuded of bone. Wear of the polyethylene cup portion of the humerus component is also visible (Nyffeler et al (2004) J Bone Joint Surg [Br] 86-B:1187-91).

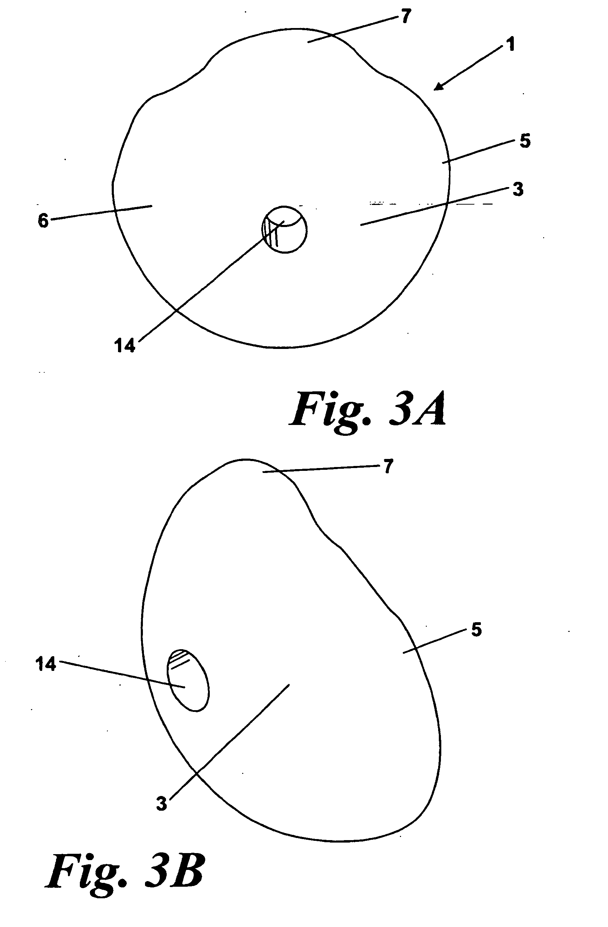

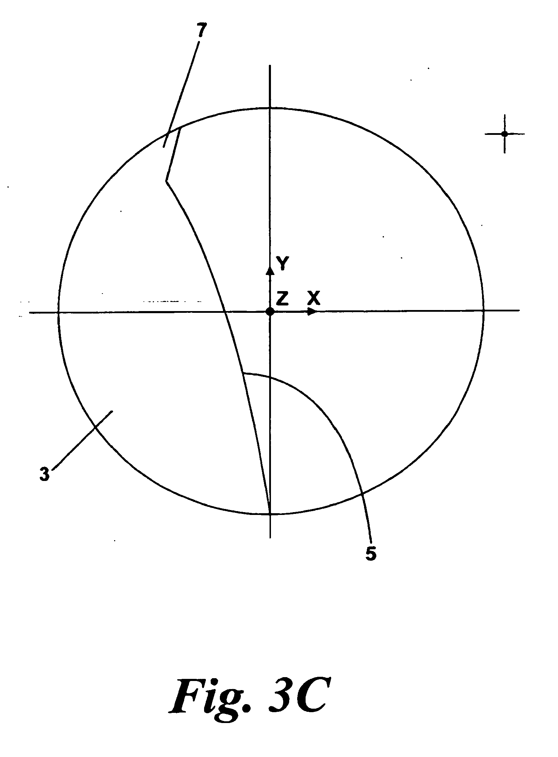

Figures 3A to 5 illustrate, in more detail, a scapula component 1 according to a first embodiment of the invention.

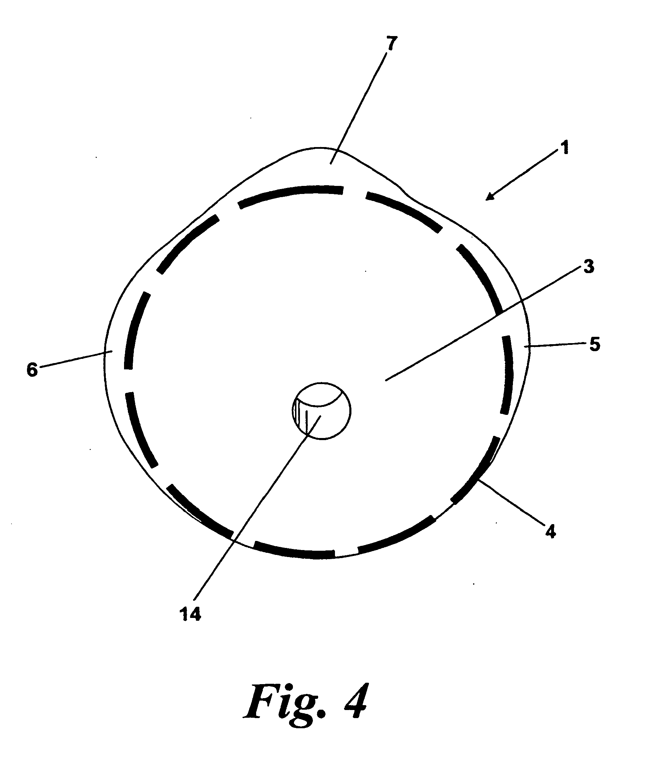

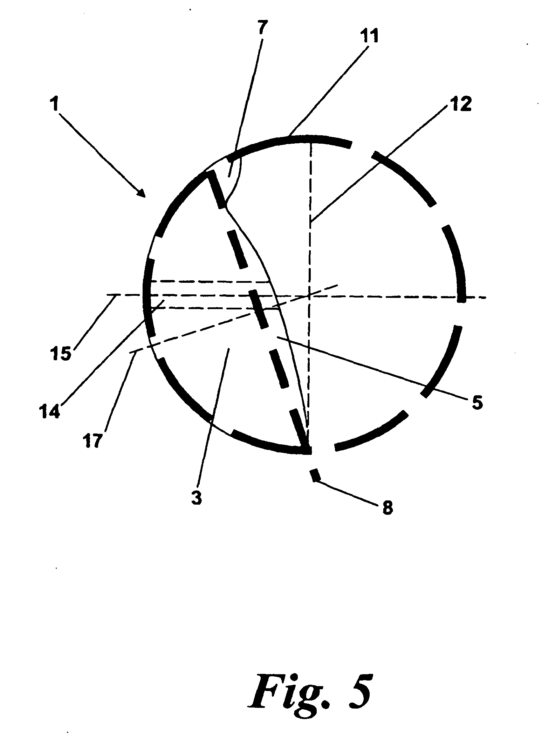

Figures 3A to 3D show a scapula component 1 according to the invention with a part spherical, convex, surface which is less than a hemisphere. The part spherical, convex, surface comprises a main surface portion 3 and three subsidiary surface portions 5, 6, 7 depending from the edge of the main surface portion 3. These portions are more clearly depicted in Figures 4 and 5. In Figure 4, a plan view of the scapula component 1, a circle 4 has been drawn onto the surface of the part spherical surface 1 to clearly illustrate the main surface portion 3. The circle 4 drawn is the largest circle that can be placed on the part spherical surface, and defines the main surface portion 3. In Figure 5, a side elevation of the scapula component 1, plane 8 defines the circular edge of the main surface portion 3.

The main surface portion 3 is circular, and the three subsidiary surface portions 5, 6, 7 which depend from the edge of the main surface portion 3 are crescent shaped. The subsidiary portions 5 and 6 are mirror images of each other and are located on opposite sides of the main surface portion 3. The crescents of subsidiary portions 5 and 6 are elongated. The third subsidiary portion 7 is located at the top, superior, edge of the main surface portion 3 and is also crescent shaped, but is less elongated than portions 5 and 6.

Preferably the subsidiary surface portions have a total surface area which is less than the surface area of the main surface portion. Preferably the total surface area of the subsidiary surface portions is less than about 90%, 80%, 70%, 60%, 50%, 40% or 30% of the surface area of the main surface portion. Preferably the width of a subsidiary portion, at its widest part, is less than about 10mm, more preferably it is less than about 5mm.

The main 3 and subsidiary 5, 6, 7 surface portions together form the part spherical surface of the scapular component 1. In use this surface forms the bearing surface that contacts the humerus component of the reverse shoulder prosthesis.

Modelling studies have shown that the subsidiary surface portions 5, 6, 7 are needed, in addition to the main surface portion 3, in order to bear the load when a reverse shoulder is in use.

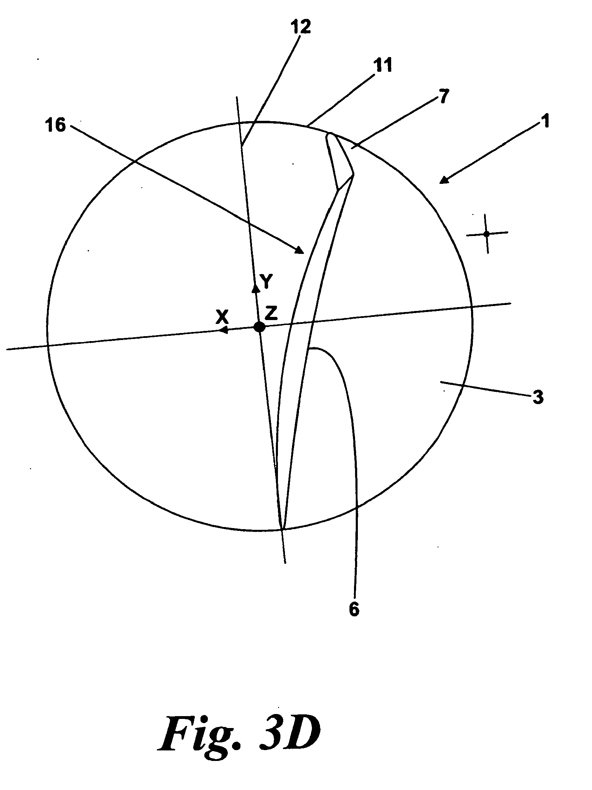

Figures 3D and 5 illustrate the difference between the scapula component 1 of the invention and a hemisphere. It is clear that the scapula component of the invention is significantly less than a hemisphere. The scapula component 1 is shown located in a hemisphere defined by lines 11 and 12. The figures illustrate that it is the base of the hemisphere, which would have reached line 12, that has been removed/is missing from the scapula component 1 when compared to a hemisphere.

The scapula component 1 also has a hole 14 though it, which extends from the main surface portion 3 of the part spherical surface of the scapular component 1 into a cavity 16 defined by the scapular component 1. The hole 14 defines a longitudinal axis indicated by the dashed line 15 in Figure 5.

The main portion 3 of the part spherical surface has a rotational axis of symmetry, illustrated by the dashed line 17 in Figure 5. The rotational axis of symmetry 17 of the main surface portion 3 of the part spherical surface is offset relative to the longitudinal axis of hole 14.

Hole 14 acts as a mounting means and allows a screw, or any other suitable fixing means to pass through the scapula component 1 to attach the component directly to the scapula or to a mounting means, in the embodiment illustrated the scapula component 1 would usually be attached to a mounting means and then to the scapula.

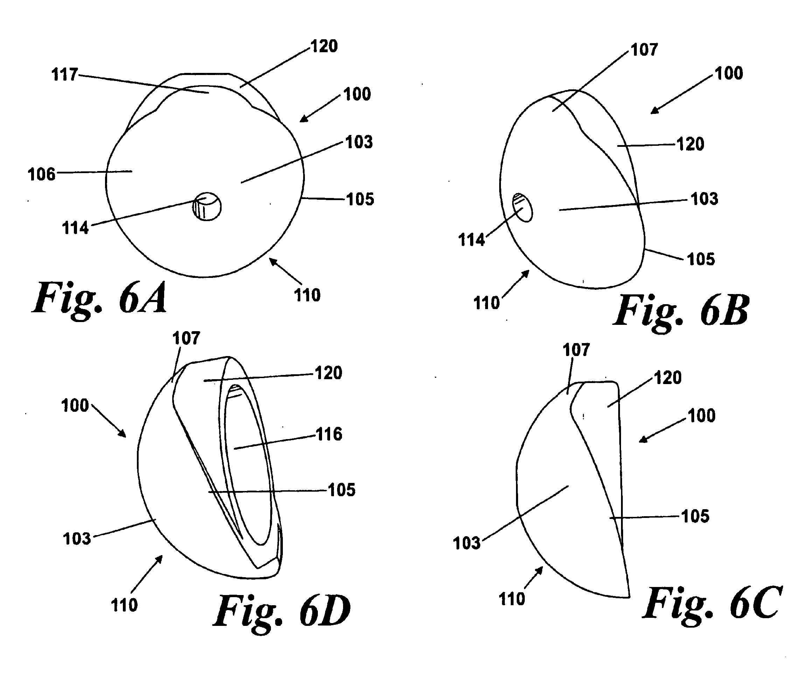

Figures 6A to Figure 8A show a second embodiment of the scapular component 100 to that depicted in Figures 3A to 5. In this embodiment the scapular component comprises a part spherical surface and a part cylindrical surface.

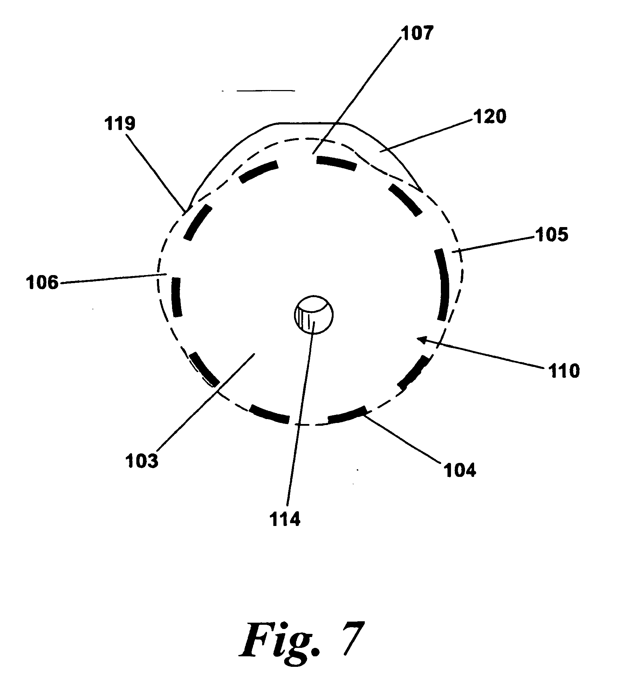

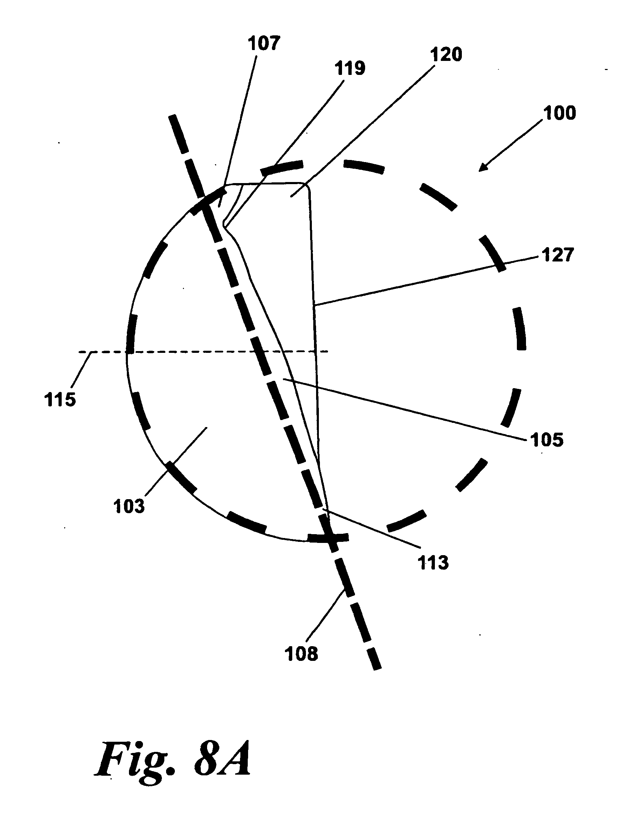

The part spherical surface is the same shape and configuration as illustrated in Figures 3A to 5 and is indicated by dotted line 119 in Figures 7 and 8A.

More specifically, the scapula component 100 comprises a part spherical, convex, surface 110 and a part cylindrical surface 120. The part spherical surface 110 is front facing and comprises a main surface portion 103 and three subsidiary surface portions 105, 106, 107 depending from the edge of the main surface portion 103. The circle 104 in Figure 7 illustrates the boundary of the main surface portion 103. The circle 104 is the largest circle that can be placed on the part spherical surface. Figure 8A shows a side elevation of the scapula component 100, in which plane 108 defines the circular edge of the main surface portion 103.

As in the first embodiment illustrated in Figures 3A to 6, the main surface portion 103 is circular, and the three subsidiary surface portions 105, 106, 107 which depend from the edge of the main surface portion 3 are crescent shaped.

In use the part spherical surface 110 forms the bearing surface that contacts the humerus component of the reverse shoulder prosthesis.

As in Figures 3A to 5 the scapula component 100 also includes a hole 114, which extends from the main surface portion 103 of the part spherical surface of the scapular component 100 into a cavity 116 defined by the scapular component 100. The hole 114 defines a longitudinal axis which is offset from the rotational axis of symmetry on the main surface portion 103.

The skilled man will appreciate that the cavity 116 may be of any suitable shape, including domed (as illustrated), cylindrical and cuboid, provided that it can be mounted to a mounting member or directly to a scapula.

In addition to the part spherical surface 110, the scapula component 100 includes a part cylindrical surface 120. The part cylindrical surface 120 extends backwards from at least a part of the back edge of the part spherical surface 110. The depth of the part cylindrical surface 120 varies along its length. The part cylindrical surface 120 extends from most of the back edge of the part spherical surface 110. The part cylindrical surface 120 may extend from between about 50% and about 100% of the back edge of the part spherical surface 110.

The part cylindrical surface 120 has a back edge 127 which is substantially flat. In use, the back edge 127 of the part cylindrical surface 120 typically contacts the scapula.

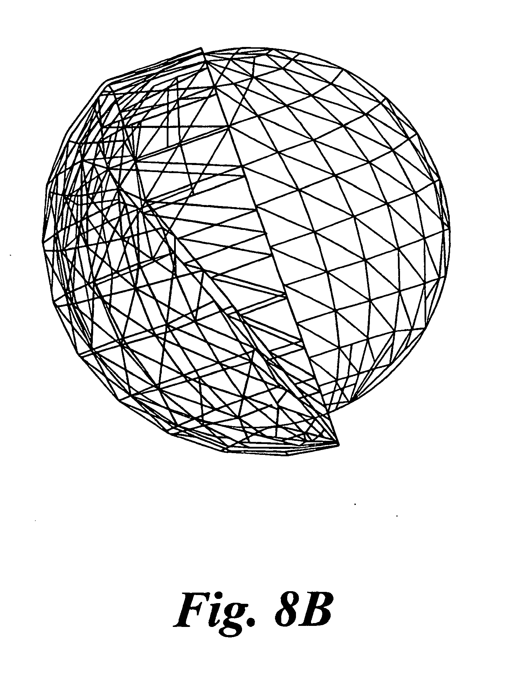

In the embodiment illustrated the back edge 127 of the part cylindrical surface 120 is a few millimetres, about 2mm, short of reaching the plane where the back surface of a hemisphere with the same radius of curvature as the part spherical surface 110 would have been. This leaves a lip 113 at the inferior edge of the part spherical surface 110. The skilled man will appreciate that this distance could be greater, or that the back surface 127 could extend to where the back surface of the hemisphere would have been as depicted in Figure 8B.

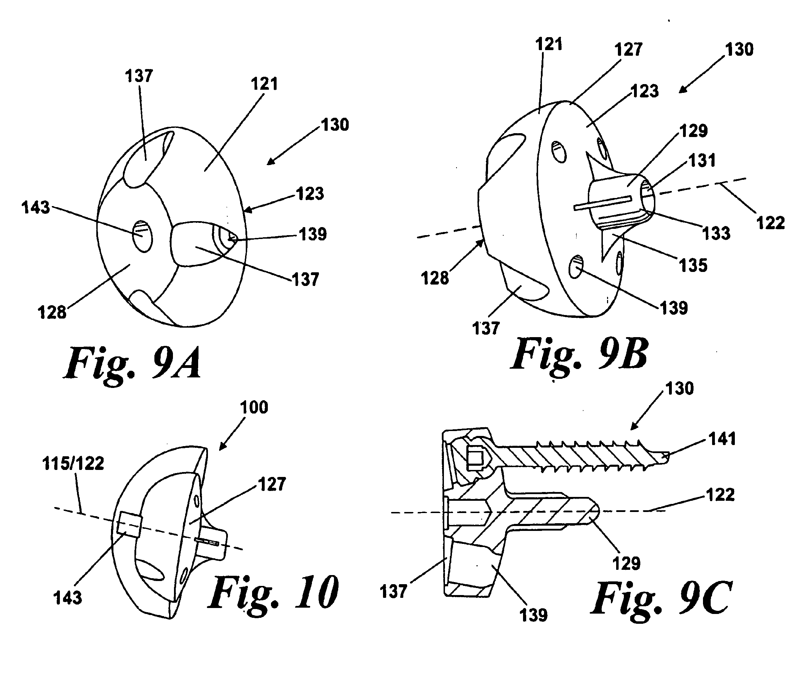

Figures 9A to 9C depict a mounting member 130 which may be used to mount the scapula component of Figures 6A to 8A to a scapula. The mounting member is arranged to be mounted onto the prepared surface of a patient's scapula (not shown) and then to engage with the cavity 116 in the scapula component 100.

Mounting member 130 is essentially domed shape. The top of the dome has been removed to leave a concave top surface 128. Sidewalls 121 slope from the top 128 to meet a base 123. The base 123 of the mounting member 130 has a convex surface 127. Preferably the convex surface 127 allows the base 123 to fit to the contours of the scapula, providing improved contact and reducing stresses. A mounting projection 129 extends from the base 23. The mounting projection 129 has a longitudinal axis 122 as shown in Figures 9A and 9C. Preferably the lohgitudinal axis 122 of the mounting projection 129 is on the same axis as the longitudinal axis 115 of the scapular component 100 when the two components are fixed together (Figure 10C). The mounting projection 129 is tubular, and the free end 131 has a chamfered edge 133. Four supporting struts 135 are equally spaced around the mounting projection 129. The mounting projection 129 may define a hole which is used to screw the mounting member 130 to a scapula. Preferably a corresponding hole is made in the patient's scapula to accommodate the mounting projection 129.

Four screw recesses 137 are equally spaced around the circumference of the mounting member 130 in the sidewalls 121 of the mounting member 130. A screw hole 139 extends from each of the screw recesses 137 through to the base 123. Each screw recess 137 is large enough to accommodate a head of a screw 141. Figure 9C shows a screw 141, as it would be positioned in a screw recess 137 and screw hole 139 when the mounting member is attached to the scapula (not shown).

A screw hole 143 is located in the centre of the flattened top 128 of the mounting member 130. The screw hole 143 has a longitudinal axis 122 in Figure 9B and 9C. This screw hole 143 extends through the centre of the mounting member 130 and through the tubular mounting projection 129. The screw hole 143 is capable of accommodating a central screw (not shown) which can secure the mounting member 130 to a patient's scapula (not shown).

The mounting member 143 may be attached to the scapula using four circumferential screws, or by using a central screw located in hole 143, or by using both means.

As an alternative to locating a screw in the central screw hole 143, the outer surface of the mounting projection 129 may be threaded and may screw directly into the scapula.

The fixing means discussed in the specific example are just one example of how the scapular component may be mounted, and the skilled man will appreciate that the device may be mounted in any suitable way. For example more or less fixing means than illustrated may be used. The fixing means may include screws, pins, nail, staples and any other suitable means.

Referring to Figure 10, screw hole 143 in the mounting member 130 is arranged to align with hole 114 in the scapula component 100, and a central screw (not shown) can be used to secure the scapula component 100 to the mounting member 130.

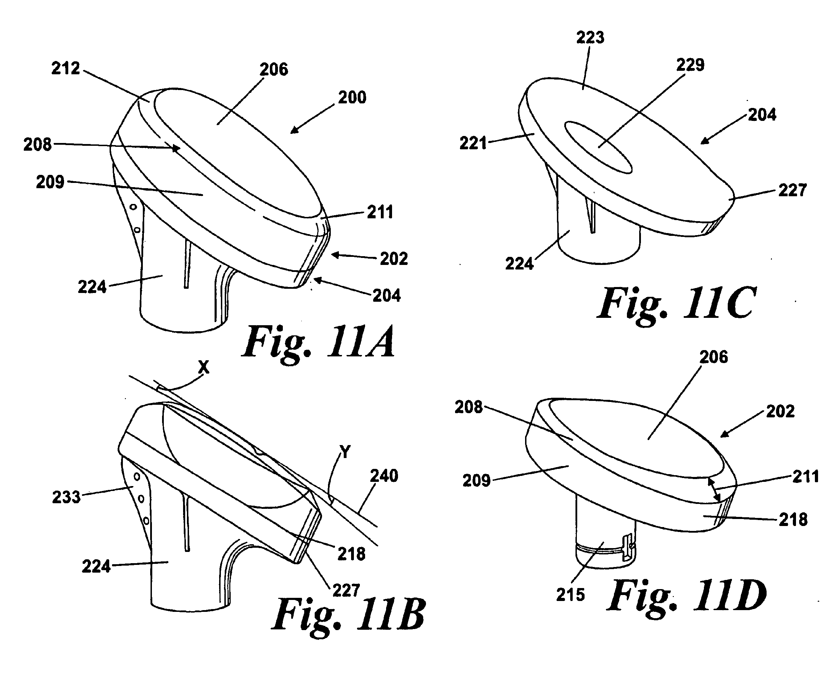

Figures 11A to 11D show a humerus component 200. The humerus component 200 comprises an upper cup portion 202 (Figure 11D) and a lower mounting portion 204 (Figure 11C).

The cup portion 202 comprises a front facing convex part spherical surface 206 surrounded by an oval shaped rim 208 with a chamfered surface. The side of the cup portion forms a cylindrical surface 209. In use, the convex surface 206 forms a bearing surface which contacts the scapula component. The underside (not shown) of the cup portion 202 is substantially flat.

The width of the rim 208 varies around the part spherical surface 206. At the lower/inferior end of the part spherical surface 206, the rim 211 is wider than at the upper/superior end of the part spherical surface 206, where the rim 212 is narrower. This difference in width of the rim is determined by how much load a particular part of the humerus component 200 will take when in use. For example, in use a lot of the load forces will be concentrated at the lower inferior end of the part spherical surface 206, where the rim 212 is wider. The wider rim 212 allows these forces to be absorbed and reduces the risk of damage to the prosthesis. Where the rim is narrower, for example at the upper end of the part spherical surface 206, the forces on the corresponding part spherical surface 206 are less and thus a narrower rim 212 can be used.

The chamfering on the rim 208 is designed to minimise impingement on the scapula which may be caused by the humerus component. As the bearing/load forces do not act directly on the surface of the rim 208, the rim can be chamfered without affecting performance of the prosthesis. Preferably the top surface on the cup portion 202 does not all lie in the same plane. As depicted in Figure 11B, the lower half of the top surface of the cup portion 202 is about 2 degrees (illustrated as angle y) from the plane 240 define by the central part of the top surface of the cup portion 202, and the upper half of the top surface of the cup portion 202 is 5 degrees (illustrated as angle x) from plane 240. The skilled man will appreciate that this is merely an example and that these angles can be varied somewhat and that the top surface may all lie in the same planar. The angling of the upper surface is intended to further minimise the risk of the humerus component impinging on the scapula.

The cup portion 202 also comprises a stem 215 which projects from the lower surface of the cup portion 202. The stem 215 is tubular and is arranged to mount the cup portion on the lower mounting portion 204.

The side 209 and the underside (not shown) of the cup portion 202 has a notch 218 at the lower most edge. This notch 218 engages with a corresponding raised portion 227 on the mounting portion 204. The notch 218 and raised portion 227 are designed to impart more strength to the lower end of the humerus component 200 where most of the load is concentrated when the component 200 is in use. The reason for this arrangement is that the lower mounting portion 204 is made of a stronger material, typically metal, which will provide the strength needed to support the load and reduce the risk of damage to the prosthesis.

The lower mounting portion 204 comprises a support portion 221, configured as a disc, with an upper surface 223 having a small spherical curvature and a lower flat surface (not shown). The small spherical curvature on the upper surface 223 reduces torsional stresses on the portion. The upper surface 223 carries a raised portion 227 which corresponds to the notch 218 on the underside of the cup portion 202.

The upper surface 223 of the humerus mounting portion 204 has a cup-portion mounting hole 229 which extends through the humerus mounting member 204 and partially into a stem 224 projecting from the underside of the support portion 221. The cup-portion mounting hole 229 engages with the cup mounting projection 215 on the cup portion 202. The hole 229 and the projection 215 can be any suitable complementary shape. They may be oval to prevent rotation.

The stem 224 projects at an angle of 60 degrees from a plane defined by the lower surface of the mounting portion 204. This angle may be varied, however the intention is that the part spherical cup of the cup portion is presented to the scapula component at an angle of about 60 degrees to the longitudinal axis of the humerus.

The stem 224 is tubular and is capable of fitting into a standard or custom anatomical stem (not shown). The stem may have any suitable cross sectional shape. The stem may be oval to prevent any rotation when in use.

The transition between the stem 224 and the lower surface of the mounting portion 204 is a smooth gradual slope. A strut 233 extends from the lower surface of the mounting portion 204 to half way along the stem 224 and serves to prevent rotation of the stem once in situ in a standard or custom anatomical stem.

## Claims

1. A scapula component (100) for a reverse shoulder prosthesis comprising a part spherical, convex, surface (110) wherein the part spherical surface does not have a rotational axis of symmetry **characterised in that** the part spherical, convex surface is less than a hemisphere of the same radius of curvature, and wherein the volume occupied by the scapula component is less than the volume of a hemisphere comprising the same radius of curvature as the part spherical portion of the scapula component.

2. A scapula component (100) according to claim 1, wherein the part spherical, convex, surface (110) comprises a main circular surface portion (103) and one or more subsidiary surface portions (105, 106, 107) depending from all or a part of the edge of the main surface portion.

3. A scapula component (100) according to claim 2, wherein the edge of the main circular surface portion (103) has at least one of the following properties: it is defined by a plane which intersects the part spherical surface (110); it is the area bounded or defined by the largest circle that will fit onto the part spherical surface; and it has a rotational axis of symmetry.

4. A scapula component (100) according to claims 2 to 3, wherein the scapula component comprises two subsidiary surfaces on opposite sides of the main circular surface portion (103) and/or a third subsidiary surface which extends from the superior edge of the main circular surface portion.

5. A scapula component (100) according to claims 2 to 4, wherein the surface area of the part spherical surface (110), or the main circular surface portion (103) thereof, is between about 5% and about 35% less than the surface area of a hemisphere with the same radius of curvature as the part spherical surface.

6. A scapula component (100) according to any preceding claim, wherein the volume occupied by the portion of the scapula component with a part spherical surface (110) is between about 5% and about 50% less than the volume that would be occupied by a hemisphere with the same radius of curvature.

7. A scapula component (100) according to any preceding claim, wherein the part spherical surface (110) defines a bearing surface which in use contacts a humerus component of a reverse shoulder prosthesis.

8. A scapula component (100) according to any preceding claim, wherein the part spherical surface (110) has a radius of curvature of between about 18 mm and about 22 mm.

9. A scapula component (100) according to any preceding claim, wherein the scapula component comprises at least one of: a mounting means to mount the scapula component either directly or indirectly to a scapula; a part cylindrical surface (120) that extends from the back of the part spherical surface (110) but does not form part of a bearing surface of the scapula component; and a mounting member (130).

10. A scapula component (100) according to claim 9, as it depends on claims 2 to 5, wherein the main surface portion (103) of the part spherical surface (110) of the scapula component has a rotational axis of symmetry that is offset at an angle from the longitudinal axis of the mounting means.

## Patentansprüche

1. Scapula-Komponente (100) für eine inverse Schulterprothese, die eine teilkugelige, konvexe Oberfläche (110) aufweist, wobei die teilkugelige Oberfläche keine Rotationssymmetrieachse aufweist, **dadurch gekennzeichnet, dass** die teilkugelige, konvexe Oberfläche weniger als eine Halbkugel des gleichen Krümmungsradius ist, und wobei das Volumen, das von der Scapula-Komponente eingenommen wird, kleiner ist als das Volumen einer Halbkugel, die den gleichen Krümmungsradius aufweist wie der teilkugelige Abschnitt der Scapula-Komponente.

2. Scapula-Komponente (100) nach Anspruch 1, wobei die teilkugelige, konvexe Oberfläche (110) einen kreisrunden Haupt-Oberflächenabschnitt (103) und einen oder mehrere Neben-Oberflächenabschnitte (105, 106, 107) aufweist, die vom gesamten Rand oder einem Teil des Randes des Haupt-Oberflächenabschnitts ausgehen.

3. Scapula-Komponente (100) nach Anspruch 2, wobei der Rand des kreisrunden Haupt-Oberflächenabschnitts (103) mindestens eine der folgenden Eigenschaften aufweist: er wird von einer Ebene definiert, welche die teilkugelige Oberfläche (110) schneidet; er ist die Fläche, die von dem größten Kreis begrenzt oder definiert wird, der in die teilkugelige Oberfläche passt; und er weist eine Rotationssymmetrieachse auf.

4. Scapula-Komponente (100) nach den Ansprüchen 2 bis 3, wobei die Scapula-Komponente zwei Neben-Oberflächen an entgegengesetzten Seiten des kreisförmigen Haupt-Oberflächenabschnitts (103) und/oder eine dritte Neben-Oberfläche aufweist, die vom oberen Rand des kreisförmigen Haupt-Oberflächenabschnitts ausgeht.

5. Scapula-Komponente (100) nach den Ansprüchen 2 bis 4, wobei der Flächeninhalt der teilkugeligen Oberfläche (110) oder von deren kreisförmigem Haupt-Oberflächenabschnitt (103) um etwa 5 % bis etwa 35 % kleiner ist als der Flächeninhalt einer Halbkugel mit dem gleichen Krümmungsradius wie die teilkugelige Oberfläche.

6. Scapula-Komponente (100) nach einem der vorangehenden Ansprüche, wobei das Volumen, das von dem eine teilkugelige Oberfläche (110) aufweisenden Abschnitt der Scapula-Komponente eingenommen wird, um etwa 5 % bis etwa 50 % geringer ist als das Volumen, das von einer Halbkugel mit dem gleichen Krümmungsradius eingenommen werden würde.

7. Scapula-Komponente (100) nach einem der vorangehenden Ansprüche, wobei die teilkugelige Oberfläche (110) eine Auflagerfläche definiert, die im Gebrauch eine Humerus-Komponente einer inversen Schulterprothese berührt.

8. Scapula-Komponente (100) nach einem der vorangehenden Ansprüche, wobei die teilkugelige Oberfläche (110) einen Krümmungsradius zwischen etwa 18 mm und etwa 22 mm aufweist.

9. Scapula-Komponente (100) nach einem der vorangehenden Ansprüche, wobei die Scapula-Komponente mindestens eines der folgenden Elemente aufweist: eine Befestigungseinrichtung, um die Scapula-Komponente entweder direkt oder indirekt an einer Scapula zu befestigen; eine teilzylindrische Oberfläche (120), die von der Rückseite der teilkugeligen Oberfläche (110) ausgeht, aber keinen Teil einer Auflagerfläche der Scapula-Komponente bildet; und ein Befestigungselement (130).

10. Scapula-Komponente (100) nach Anspruch 9, soweit dieser von einem der Ansprüche 2 bis 5 abhängt, wobei der Haupt-Oberflächenabschnitt (103) der teilkugeligen Oberfläche (110) der Scapula-Komponente eine Rotationssymmetrieachse aufweist, die winkelversetzt ist zur Längsachse der Befestigungseinrichtung.

## Revendications

1. Un composant de scapula (100) pour une prothèse d'épaule inversée comprenant une surface convexe partiellement sphérique (110) où la surface partiellement sphérique ne possède pas un axe de symétrie en rotation, **caractérisé en ce que** la surface convexe partiellement sphérique est inférieure à une hémisphère du même rayon de courbure, et où le volume occupé par le composant de scapula est inférieur au volume d'une hémisphère comprenant le même rayon de courbure que la partie partiellement sphérique du composant de scapula.

2. Un composant de scapula (100) selon la Revendication 1, où la surface convexe partiellement sphérique (110) comprend une partie de surface circulaire principale (103) et une ou plusieurs parties de surface subsidiaire (105, 106, 107) qui dépendent de tout ou partie de la bordure de la partie de surface principale.

3. Un composant de scapula (100) selon la Revendication 2, où la bordure de la partie de surface circulaire principale (103) possède au moins l'une des propriétés suivantes : elle est définie par un plan qui coupe la surface partiellement sphérique (110), elle est la zone limitée ou définie par le cercle le plus grand qui pourra s'insérer dans la surface partiellement sphérique et elle possède un axe de symétrie en rotation.

4. Un composant de scapula (100) selon les Revendications 2 à 3, où le composant de scapula comprend deux surfaces subsidiaires sur des côtés opposés de la partie de surface circulaire principale (103) et/ou une troisième surface subsidiaire qui s'étend à partir de la bordure supérieure de la partie de surface circulaire principale.

5. Un composant de scapula (100) selon les Revendications 2 à 4, où la superficie de la surface partiellement sphérique (110), ou la partie de surface circulaire principale (103) de celle-ci, représente entre environ 5% et environ 35% de moins que la superficie d'une hémisphère avec le même rayon de courbure que la surface partiellement sphérique.

6. Un composant de scapula (100) selon l'une quelconque des Revendications précédentes, où le volume occupé par la partie du composant de scapula avec une surface partiellement sphérique (110) représente entre environ 5% et environ 50% de moins que le volume qui serait occupé par une hémisphère avec le même rayon de courbure.

7. Un composant de scapula (100) selon l'une quelconque des Revendications précédentes, où la surface partiellement sphérique (110) définit une surface d'appui qui, en utilisation, entre en contact avec un composant d'humérus d'une prothèse d'épaule inversée.

8. Un composant de scapula (100) selon l'une quelconque des Revendications précédentes, où la surface partiellement sphérique (110) possède un rayon de courbure situé entre environ 18 mm et environ 22 mm.

9. Un composant de scapula (100) selon l'une quelconque des Revendications précédentes, où le composant de scapula comprend au moins un élément parmi : un moyen de montage destiné à monter le composant de scapula soit directement ou indirectement sur une scapula, une surface partiellement cylindrique (120) qui s'étend à partir de l'arrière de la surface partiellement sphérique (110) mais ne forme pas une partie d'une surface d'appui du composant de scapula, et un élément de montage (130).

10. Un composant de scapula (100) selon la Revendication 9, lorsqu'elle dépend des Revendications 2 à 5, où la partie de surface principale (103) de la surface partiellement sphérique (110) du composant de scapula possède un axe de symétrie en rotation qui est décalé d'un angle à partir de l'axe longitudinal du moyen de montage.
